(19) Europäisches Patentamt
European Patent Office
Office européen des brevets

(11) Publication number: 0 252 738 A2

(12) # EUROPEAN PATENT APPLICATION

(21) Application number: 87306074.3

(22) Date of filing: 09.07.87

(51) Int. Cl.$^4$: G 01 N 33/22
G 01 N 33/26, G 01 N 9/00

(30) Priority: 11.07.86 GB 8617001

(43) Date of publication of application:
13.01.88 Bulletin 88/02

(84) Designated Contracting States:
BE FR GB IT NL SE

(71) Applicant: BP Chemicals Limited
Belgrave House 76 Buckingham Palace Road
London, SW1W 0SU (GB)

The British Petroleum Company p.l.c.
Britannic House Moor Lane
London EC2Y 9BU (GB)

(72) Inventor: Psaila, Alexander Francis
BP Chemicals Limited Sully Penarth
South Glamorgan CF6 2YU (GB)

Hickman, Stephen Anthony
The British Petroleum Company p.l.c. Chertsey Road
Sunbury-on-Thames Middlesex TW16 7LN (GB)

Lawrence, Frank Timothy
The British Petroleum Company p.l.c. Chertsey Road
Sunbury-on-Thames Middlesex TW16 7LN (GB)

(74) Representative: Krishnan, Suryanarayana Kalyana et al
BP INTERNATIONAL LIMITED Patents & Agreements
Division Chertsey Road
Sunbury-on-Thames Middlesex TW16 7LN (GB)

(54) **Foam meter.**

(57) This invention relates to a foam meter which has a thermostatically controlled column open at one end with a mesh at the other end. The mesh is of a uniform pore size and gas is sparged through the pores onto a liquid placed on the mesh whereby the liquid foams. The volume of foam generated is measured by graduations on the column. The meter is used for measuring the foamability of crude oil and to determine the performance of antifoams used during crude oil recovery.

EP 0 252 738 A2

**Description**

FOAM METER

The present invention relates to a foam meter used for the characterisation of the foaming tendency of liquids and the performance of antifoam formulations.

Accurate measurement of the performance of antifoam formulations to evaluate their ability to disperse foams is important. This is particularly so in the case of antifoams used to minimise foaming in crude oils, sugar solutions, beer etc. Thus screening of antifoam formulations must meet two important objectives:

(a) rank candidate chemicals into performance groups e.g. good, poor and mediocre, and

(b) point to new areas of synthetic work by establishing structure performance relationships.

Also the choice of a suitable screening test is determined by three considerations, namely, the complexity of the test method; its reproducibility and repeatability and finally, its relevance to actual user performance.

Traditional methods used for the study of foams employ one of three ways of generating a foam, namely, single capillaries (e.g. Stig. E. Fribergy and J. Mark Cox, Chemistry and Industry, 17th January 1981, page 50), sintered glass spargers (e.g. J.J. Bikerman, Transactions of the Faraday Society, Vol 34, pagaes 634-638), and diffuser stones (e.g. IP 146/82, Standard Method of Test for Foaming Characteristics of Lubricating Oils). Single capillaries give uniform bubbles but are relatively slow. The other methods generate a rapid foam with considerable turbulence, which adversely affects the repeatability of results. It also became apparent that glass spargers when used to study crude oil foams are prone to gradual clogging by debris in the oil.

The present invention deals with a method for generating a turbulence free, uniform bubble-sized foam rapidly and reproducibly. Furthermore the results obtained using this method are independent of the actual hardware employed.

Accordingly, the present invention is a foam meter comprising:

(a) a thermostatically controlled foam column open at one end and containing a mesh having a substantially uniform pore size of an area greater than 0.0025 $mm^2$,

(b) means for sparging a gas into the column through the mesh at a predetermined temperature and pressure, and

(c) means for measuring the volume of foam generated upon sparging the gas through a liquid under test placed on the mesh in the column.

One of the essential features of the invention is that the pores in the mesh have to be of a substantially uniform size. By this is meant that not more than 5%, preferably not more than 2.5% of the total number of pores in the mesh should deviate from the average pore size specified.

For laboratory tests, the foam column is suitably made of glass and is fitted with a wire mesh suitably of stainless steel. The uniform pore size of the mesh will depend upon the liquid being tested, the only criterion being that no significant amount of the liquid under test should pass through the mesh against the flow of the sparging gas during testing. The pore size of the mesh is suitably from 0.003 $mm^2$ to 0.03 $mm^2$, preferably from 0.01 $mm^2$ to 0.03 $mm^2$. The column is suitably graduated to enable a direct reading of the foam volume from the column.

The density of uniform holes in the mesh is such that there is not too much dead space on the mesh. Thus, in a mesh 125 micrometers x 125 micrometers there are at least 50-100 pores.

The mesh is supported on a funnel shaped conduit to enable a filler gas to be passed uniformly through the mesh. The mesh can be constructed from woven wire or from a perforated plate of stainless steel or of any other suitable material, e.g. polytetrafluoroethylene (PTFE). Uniform dimension of the perforations is critically important for the formation of a foam structure free of turbulence. One defective hole which is significantly oversized with respect to the rest in the mesh is sufficient to cause instability in the foam as this creates a preferred passage for the gas streaming through the liquid.

In order to measure the foaming characteristics of a given liquid e.g. crude oil, the flow of gas through the foam meter is first established and then a sample of the liquid is placed in the column just above the mesh.

The pressure of the gas being sparged or used as a filler is controlled by a needle valve and the mesh. The rate of flow of sparging gas is measured by a calibrated flow meter and is presaturated prior to use by passing over a sample of the liquid being tested in a dreschel bottle. If it is desired to carry out the foaming test at a given temperature, this may be achieved by heating the sparging gas to a predetermined temperature before contact with the mesh or the liquid being foam tested. Thus, a coil capable of being heated to a predetermined temperature may be positioned between the mesh and the gas flow meter.

The temperature of the contents of the foam column may be maintained constant by any of the well known thermostatic control means. For instance, a water jacket may be used to surround the column.

When the liquid to be foam tested is placed on the mesh, a steady flow of gas under controlled pressure is applied to the mesh to prevent the liquid being tested from flowing into or through the mesh. Thereafter the pressure of the gas is gradually increased to allow the gas to be sparged or aspirated through the mesh but avoiding any turbulence. The flow of the sparging gas can be controlled by a three way valve placed between the coil and the mesh. The three way valve also enables the column to be drained after each measurement is completed.

The measurements are carried out by monitoring the foam volume of a liquid before and after the addition of an antifoam at a constant flow rate of the sparging gas. In the case of a transparent glass tube being used as

the column, the tube may be graduated and the foam volume monitored visually through the column.

The equilibrium foam height before injection and after injection of the antifoam can be recorded and the efficiency of the antifoam or the so-called "defoaming index" (DFI) can be calculated using the equation:-

$$DFI = \frac{\text{Foam volume (no AF) - Foam Volume (+AF)}}{\text{Foam volume (no AF) - Liquid volume (no foams)}}$$

$$= \frac{\text{Foam height (no AF) - Foam height (+AF)}}{\text{Foam height (no AF) - Liquid height (no foam)}}$$

wherein AF represents antifoam additive.

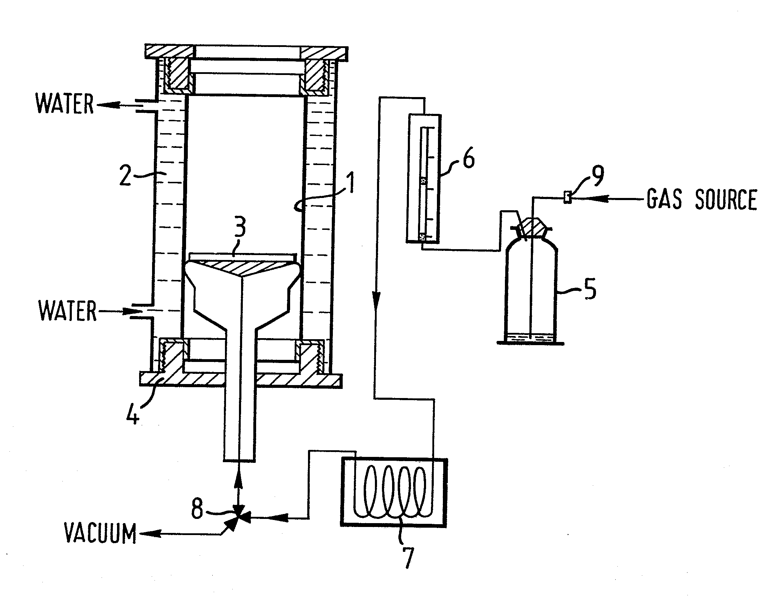

The foam meter of the present invention is further illustrated with reference to the accompanying drawing.

In the drawing a column (1) having graduations thereon (not shown) is provided with a water jacket (2) and a wire mesh (3) in sealing engagement with the interior of the column. The wire mesh (3) is detachable from the column and is retained on the column by means of a screw-on unit (4). A dreschel bottle (5) is provided to purify the sparging gas supplied by a source (not shown) prior to use.

A flow meter (6) is provided to monitor the flow of the sparging gas from the dreschel bottle (5) into the mesh (3) and hence the column (1). A coil (7) is also provided which is capable of heating the sparging gas at a predetermined temperature prior to entry into the column.

A three way valve (8) is positioned between the coil (7) and the mesh (3) to enable (a) the flow of sparging gas into the mesh to be controlled and (b) drainage the column after each measurement is completed. Gas flow rate can be varied by means of the needle valve (9).

In operation, a sample of the liquid is placed in the column (1) on the mesh (3) after the sparger gas flow at a predetermined temperature and flow rate is initiated so that the sample liquid does not flow through the mesh (3). The temperature of the column is maintained constant by the water jacket (2). When equilibrium is reached, the height of the foam in the column is measured by graduations thereon (not shown) and thus foam height is recorded. Thereafter, an antifoam agent is injected into the foamed liquid by means of a microlitre syringe and the contents of the column are again allowed to attain equilibrium and the new foam height recorded. From the difference between the two foam heights with and without the antifoam and from the initial volume of the liquid sample, the defoaming index (DFI) is calculated according to the equation above. Thereafter the column was drained and evacuated through the three way valve (8).

The present invention is further illustrated with reference to the following Examples.

General Procedure

Methane gas at 25°C was sparged through stabilised crude oil (10 $cm^3$) in a 45 cm long thermostated tube (2.5 cm diameter). The foaming crude oil was supported on a coarse mesh (125 microns x 125 microns) by the pressure of the filler gas.

Aspiration of methane gas through the mesh generated a foam of uniform bubble size and substantially free of turbulence.

Gas velocity was measured using a calibrated rotameter or a wet gas meter and stop watch.

The performance of antifoam was monitored by measurement of foam volume before and after addition of the chemical. The antifoam was introduced into the foaming crude by means of a long (20 cm) needle fitted to a microlitre syringe.

Example 1

Effect of mesh size on Foam Index (or Foam lifetime) of a stabilised crude oil.

Two foam meters identical in every respect, except for the dimensins of the wire mesh, were used to determine the foam volume-gas flow rate relationship for Magnus stabilized crude (10 $cm^3$ at 25°C). The results are summarised in Table 1. Essentially linear relationships are obtained, and in both cases the slope of each graph is identical (foam life time).

$$\text{Foam life time} = \frac{\text{Foam Volume}}{\text{Gas Flow Rate}}$$

TABLE 1

| | Foam Height/cm | |
|---|---|---|
| Gas Flow rate/cm$^3$ | Coarse Mesh | Fine Mesh |
| 100 | 5.2 | 7.0 |
| 120 | 7.5 | |
| 140 | 9.8 | 11.5 |
| 170 | 13.0 | |
| 180 | | 15 |
| Foam lifetime /s | 32 | 30 |
| Mesh size/mm$^2$ | 0.0156 | 0.0121 |

This example illustrates that the measurement of foam lifetime, a characteristic of the liquid and gas system being studied, is independent of the equipment used despite the significant changes in mesh size used in the two cases. This was achieved because mechanical factors which contribute to foam instability (chiefly turbulence and non-uniform bubbles) have been eliminated.

Example 2

In this Example the foam meter is used as a quality control tool. The performance of compound B is compared with the reference material A. The defoaming ability of both materials was measured at dose levels of 1.0, 0.5 and 0.1 ppm. Each measurement is performed in triplicate using three freshly prepared samples.

The defoaming power is expressed as an index: DI

$$DI = \frac{\text{Foam height (max)} - \text{Foam height (min)}}{\text{Foam height (max)} - \text{Liquid height (no frame)}}$$

The results are summarised in Table 2. At each concentration the spread of DI is an indication of the reproducibility of the test method. If this is considered satisfactory then judgement of the acceptability of the new compound 'B' can be made by comparison of the 'average' DI at each dose level. In this Example compound B was considered to be satisfactory.

Concentration of AF2001 = 1 ppm

| Sample | Foam Ht max (cm) | Foam Ht min (cm) | DI | Average | Spread |
|---|---|---|---|---|---|
| A1 | 13.0 | 2.4 | 0.94 | | |
| A2 | 15.0 | 2.7 | 0.93 | 0.92 | +/-.02 |
| A3 | 15.2 | 2.9 | 0.91 | | |
| B1 | 15.3 | 2.7 | 0.93 | | |
| B2 | 15.4 | 2.8 | 0.92 | 0.92 | +/-.01 |
| B3 | 15.4 | 2.8 | 0.92 | | |

Concentration of AF2001 = 0.5 ppm

| Sample | Foam Ht max (cm) | Foam Ht min (cm) | DI | Average | Spread |
|---|---|---|---|---|---|
| A11 | 15.0 | 5.8 | 0.69 | | |
| A21 | 15.2 | 5.8 | 0.70 | 0.69 | +/-0.1 |
| A31 | 15.0 | 5.8 | 0.69 | | |
| B11 | 15.2 | 5.7 | 0.70 | | |
| B21 | 15.2 | 5.6 | 0.70 | 0.71 | +/-0.1 |
| B31 | 15.0 | 5.4 | 0.72 | | |

Concentration of AF2001 = 0.1 ppm

| Sample | Foam Ht max (cm) | Foam Ht min (cm) | DI | Average | Spread |
|---|---|---|---|---|---|
| A11 | 15.3 | 8.5 | 0.50 | | |
| A21 | 15.0 | 8.7 | 0.47 | 0.46 | +/-.04 |
| A31 | 15.1 | 8.8 | 0.42 | | |
| B11 | 15.5 | 8.6 | 0.50 | | |
| B21 | 15.4 | 8.6 | 0.49 | 0.49 | +/-.01 |
| B31 | 15.2 | 8.5 | 0.49 | | |

Example 3

The foam meter can be used to study the foam life time dependence of temperature. Small changes in temperature can alter the foaming properties of a liquid quite significantly. In this Example the foam life time of stabilised Brae crude was determined at 25°C and 35°C. 10 $cm^3$ of stabilised Brae crude was sparged with natural gas.

(a) at 25°C

| Gas Flow Rate/$cm^3 min^{-1}$ | Foam Height/cm |
|---|---|
| 290 | 13 |
| 250 | 10.3 |
| 230 | 8.3 |
| 196 | 6.2 |
| 180 | 4.4 |

(b) at 35°C

| Gas Flow Rate/$cm^3 min^{-1}$ | Foam Height/cm |
|---|---|
| 274 | 9.1 |
| 254 | 8.4 |
| 237 | 7.5 |
| 227 | 6.8 |
| 208 | 6.0 |
| 193 | 4.8 |
| 170 | 3.6 |

Foam life time at 25°C/s = 23
Foam life time at 35°C/s = 16

Example 4

In this Example the foam meter was used to evaluate the performance of a series of antifoam candidates of varying molecular weight. A silicone glycol antifoam of this type is

$$(CH_3)_3\ Si\ O\ (CH_2\ Si\ O)_x - (CH_2-\underset{R}{\underset{|}{Si}}\ O)_y\ Si\ (CH_3)_3$$

where R is an allyl substituted polyethylene glycol of molecular weight 350. In this study the ratio of x/y was kept constant (8) and x + y varied. Ninian crude oil (10 $cm^3$) at 25°C was sparged with 110 $cm^3\ min^{-1}$ of natural gas. The foam was treated with 0.3 ppm of the antifoam. The defoaming index (DI) was determined.

| x + y | Di |
|---|---|
| 10 | 0.09 |
| 25 | 0.80 |
| 50 | 0.95 |
| 75 | 0.96 |
| 100 | 0.96 |
| 150 | 0.95 |

**Claims**

1. A foam meter comprising:

(a) a thermostatically controlled foam column open at one end and containing a mesh having a substantially uniform pore size of an area greater than 0.0025 $mm^2$,

(b) means for sparging a gas into the column through the mesh at a predetermined temperature and pressure, and

(c) means for measuring the volume of foam generated upon sparging the gas through a liquid under test placed on the mesh in the column.

2. A foam meter according to claim 1 wherein the uniform pore size of the mesh is from 0.003 $mm^2$ to 0.03 $mm^2$.

3. A foam meter according to claim 1 or 2 wherein the mesh is supported on a funnel shaped conduit.

4. A foam meter according to any one of the preceding claims wherein the mesh is constructed from woven wire, or from a perforated plate of stainless steel or polytetrafluoroethylene.

5. A foam meter according to any one of the preceding claims wherein a coil is provided which is capable of heating the sparging gas to the predetermined temperature prior to contact with the mesh.

6. A method of measuring the volume of foam generated by a liquid upon sparging a gas therethrough comprising (a) placing the liquid under test on a mesh in a thermostatically controlled foam column which is open at one end and contains a mesh, the mesh having a substantially uniform pore size of an area greater than 0.0025 $mm^2$, (b) sparging a gas into the column through the mesh and the liquid thereon at a predetermined temperature and pressure, and (c) measuring the volume of the foam in the column resulting from the sparging of the liquid by the sparging gas.

0252738